Europäisches Patentamt

European Patent Office

Office européen des brevets

(19)

(11) Publication number:    **0 063 034**
A2

# (12) EUROPEAN PATENT APPLICATION

(21) Application number: **82301847.8**

(22) Date of filing: **07.04.82**

(51) Int. Cl.³: **C 01 F 7/00**
**C 01 F 7/36, B 01 J 13/00**
**C 04 B 35/00**

(30) Priority: **10.04.81 GB 8111389**
**22.10.81 GB 8131860**

(43) Date of publication of application:
**20.10.82 Bulletin 82/42**

(84) Designated Contracting States:
**AT BE CH DE FR GB IT LI LU NL SE**

(71) Applicant: **ZIRCONAL PROCESSES LIMITED**
**Cosmos House Bromley Common**
**Bromley Kent BR2 9TL(GB)**

(72) Inventor: **Emblem, Harold Garton**
**6 Pratt Lane**
**Mirfield West Yokshire WF14 9LX(GB)**

(72) Inventor: **Jones, Kenneth**
**17 St. Austell Avenue**
**Tyldesley Manchester(GB)**

(72) Inventor: **Parkes, Peter**
**12 Wise Street**
**Dresden Stoke-on-Trent ST3 4PQ(GB)**

(74) Representative: **Daley, Michael John et al,**
**F.J. CLEVELAND & COMPANY 40/43 Chancery Lane**
**London, WC2A 1JQ(GB)**

(54) Rigid coherent gel.

(57) A rigid coherent gell for binding refractories and other purposes is made by hydrolysing an aluminium alkoxide or aroxide and causing or allowing the hydrolysate to set.

EP 0 063 034 A2

COMPLETE DOCUMENT

Croydon Printing Company Ltd.

- 2 -

## DESCRIPTION

This invention relates to the production of a rigid coherent gel from a settable liquid.

According to the present invention an aluminium alkoxide may be used as the precursor of a rigid coherent gel suitable for binding refractory grains. Rigid coherent gels based on an aluminium alkoxide or aroxides can also be used as catalysts or as supports for catalysts, also as adsorbents or molecular sieves, also as fillers for plastic mouldings.

The aluminium alkoxide may be hydrolysed to a gellable hydrolysate under acidic, neutral or basic conditions. Hydrolysis under basic conditions is convenient. An amine hydrolysis catalyst may be used. Desirably the amine is a strongly basic aminoalcohol, for instance monoethanolamine, diethanolamine or triethanolamine, or a mixture of these aminoalcohols. Under these conditions, hydrolysis and gelation occurs at the same time. If the hydrolysis is carried out under acidic or neutral conditions, gelation can be induced by heating the system, or by treatment with a base, for example with an amine or an aminoalcohol.

Usually a mutual solvent will be required for the hydrolysis. The mutual solvent is conveniently a $C_1 - C_3$ alcohol, for example isopropanol.

Refractory shapes may be prepared according to the invention by pouring the slurry formed from a refractory powder and an aluminium alkoxide hydrolysate into a suitable mould, causing or allowing the slurry to set, then removing the cast shape from the mould, drying the shape and firing it to obtain a refractory object. This procedure can be used to make special refractories suitable for use in the glass and metallurgical industries. Examples are orifice rings, also nozzles and well-blocks to be used in sliding gate systems for steel casting. The refractory shape may also be a mould, part-mould or core to be used in casting molten metals and alloys.

Suitable aluminium alkoxides can be represented by the formula $Al(OR)_3$, or co-ordination polymers thereof, i.e. $(Al(OR)_3)N$, N usually being 2,3 or 4. R is an alkyl group, linear or branched or an aromatic group. The R groups may be the same or different, or may be cyclo-alkyl groups. Preferably the alkyl groups do not contain more than six carbon atoms. Other suitable aluminium alkoxides include those containing the grouping Al-O-Al, also those containing acyloxy groups -OCOR' , in which R' is an alkyl group, linear, branched or cyclo-alkyl and may, if desired, be unsaturated. Mixtures of various aluminium alkoxides can also be used. Specifically aluminium alkoxides which are convenient to use include aluminium isopropoxide and the aluminium butoxides. Another convenient aluminium alkoxide is a mixed aluminium isopropoxide secbutoxide. These aluminium alkoxides may, if desired, be used in solution in white spirit.

The invention further contemplates the use, as a precursor of a rigid coherent gel of a mixed alkoxide of aluminium and another metal such as magnesium. Mixtures of aluminium and other metal alkoxides are also envisaged. Indeed the silica aquasol system referred to above may be regarded as a mixed alkoxide.

The following are Examples of the invention:-

## EXAMPLE I

5 ml of Manalox 30A (a solution of aluminium tri-isopropoxide modified with a higher molecular weight alcohol) and 1.5 ml triethanolamine were mixed and to this mixture was added 2.5 ml water in 5 ml isopropanol. This provides a gellable hydrolysate with alkaline gellation catalyst constituting the binder solution. The binder solution was added to 125 grams "Sardamag" magnesia grain mix to give a slurry which was cast into a mould. The casting was removed from the mould after 20 minutes. The binder solution gives a rigid strong gel in 12 minutes, increasing in strength.

EXAMPLE II

10 ml of Manalox 30A and 2.6 ml triethanolamine were mixed and to this mixture was added 5 ml water in 15 ml isopropanol. This gives the binder solution. 5 ml of the binder solution was added to 50 grams "Sardamag" magnesia grain to give a slurry which was cast into a mould.

EXAMPLE III

10 ml Manalox 30A and 2.6 ml triethanolamine were mixed and to this mixture was added 5 ml water in 12 ml isopropanol. This gives the binder solution. The binder solution was added to 225 grams of "Sardamag" magnesia grain mix, to give a slurry which was cast into a mould. The casting was removed from the mould after 20 minutes. Compared with Example I, this casting had an increased rate of development of strength.

EXAMPLE IV

20 ml Manalox 205, which is a polymeric aluminium acetate/isopropoxide containing about 25% of the solvent ester isopropyl acetate, was mixed with 225 grams Sardamag magnesia grain mix, then a mixture comprising 6 ml triethanolamine, 2 ml water and 6 ml isopropanol was added. The resulting slurry was cast into a mould. The setting time of the slurry was about $3\frac{1}{2}$ minutes.

EXAMPLE V

A mixture of 20 ml Manalox 205 and 6 ml triethanolamine was mixed with 225 grams "Sardamag" magnesia grain and the resulting slurry cast into a mould. After one hour, the casting was removed from the mould.

EXAMPLE VI

10 ml Manalox Aliso B (a mixed isopropoxide and secondary butoxide of aluminium) and 3 ml triethanolamine were mixed (caution - this is exothermic), to this mixture was added 5 ml water and 10 ml isopropanol. This gives the binder solution, which gels in about 2 minutes. The binder solution was added to 225 grams "Sardamag" magnesia grain mix, to give a slurry which was cast into a mould. This gave a good casting, but the slurry was too wet.

EXAMPLE VII

The binder solution was prepared as in Example VI. 20 ml of the binder solution was added to 200 grams "Sardamag" magnesia grain mix to give a slurry which was cast into a mould. After 6 minutes, the casting was removed from the mould (too soon, casting only just strong enough to remove from mould).

<u>Composition of Sardamag grain mix used in above</u>
<u>Examples</u>

| B.S.410; 1976<br>Sieve number (and<br>aperture | | Percentage by Weight |
|---|---|---|
| - 5 + 10 | (-3.35mm + 1.70mm) | 30 |
| - 10 + 25 | (-1.70mm + 600 µm) | 20 |
| - 25 + 72 | (-600 µm + 212µm) | 15 |
| Ball-milled fines | | 35 |

The Sardamag grain used was Sardamag 251 SP grade.

The ball-milled fines had the following properties:-

| | | |
|---|---|---|
| $Al_2O_3$ | - | 0.25% by weight |
| $Fe_2O_3$ | - | 0.22% by weight |
| CaO | - | 1.62% by weight |
| $SiO_2$ | - | 0.78% by weight |

Residue MgO

BET surface area $2m^2/g$ (approx.)

Surface area by Rigden method 0.25 - $0.30m^2/g$.

Sardamag 251 SP grain has the following

typical analysis:

| MINERAL | WEIGHT PERCENT |
|---------|----------------|
| $SiO_2$ | 0.7 - 0.9 |
| $Al_2O_3$ | 0.2 - 0.4 |
| $Fe_2O_3$ | 0.15 - 0.3 |
| $B_2O_3$ | 0.02 - 0.4 |
| CaO | 1.8 - 2.3 |
| MgO | 95.5 - 96.5 |

The firing procedures for the "Sardamag" magnesia based castings are those normally followed for firing magnesia refractories.

EXAMPLE IX

Magnesium and the aluminium alkoxide mixture (Manalox Aliso B) react in isopropanol to give a mixed double alkoxide of magnesium and aluminium.

200 g Manalox Aliso B were dissolved in 200 ml isopropanol (dried over a molecular sieve). 15 g magnesium powder were added to the solution, which was refluxed for ca 56 hours, about 0.5 g mercuric chloride being added as catalyst at 8-hourly intervals. The double alkoxide was purified by distillation (140°C at 0.1 mm Hg), yield ca 76% after distillation.

5 ml of the double alkoxide and 1 ml triethanolamine were mixed (caution - exothermic) and to this mixture was added a mixture of 1.8 ml water in 2.2 ml isopropanol. A rigid coherent gel was obtained in about 20 minutes.

EXAMPLE X

3 ml triethanolamine were added to 15 ml double alkoxide of magnesium and aluminium prepared as described in Example IX (this is strongly exothermic). To this mixture 225 g Sardamag magnesia grain were added, followed by a mixture of 5.4 ml water in 8.4 ml isopropanol. The slurry was cast into a mould, the casting being removed from the mould after 30 minutes, giving a strong article whose strength improved on air-drying. The Sardamag grain composition was as used in the previous examples.

EXAMPLE XI

3 ml triethanolamine were added to 15 ml of the double alkoxide of magnesium and aluminium prepared as described in Example IX (caution - exothermic). To this mixture 225 grams of alumina grain mix were added, followed by a mixture of 5.4 ml water in 6.6 ml isopropanol. The slurry was cast into a mould, the casting being removed from the mould after about 20 minutes giving a strong article.

The composition of the alumina grain mix was:

| Tabular Alumina Grain B.S.410 Screen or Mesh No. | Percentage by Weight |
|---|---|
| $-\frac{1}{4}$ " + 8 | 6 |
| - 8 + 14 | 24 |
| -14 + 25 | 31 |
| -25 + 48 | 7 |
| -48 | 8 |
| -100 | 7 |
| BACO MA 95 | 17 |
| Calcined Alumina | |

EXAMPLE XII

The Table gives conditions under which rigid coherent gels can be obtained from the double alkoxide of magnesium and aluminium, whose preparation is given in Example IX. The triethanolamine and the double alkoxide are mixed, then to this mixture is added the mixture of water and isopropanol.

| Volume (ml) of Double Alkoxide | Volume (ml) of Triethanolamine | Volume of Water (ml) | Volume of Isopropanol | Gel Time |
|---|---|---|---|---|
| 5 | 1 | 1.9 | 2.1 | 1.5 min |
| 5 | 0.9 | 1.4 | 2.7 | 25 min |
| 5 | 1 | 1.8 | 2.2 | 20 min |
| 5 | 1 | 1.85 | 2.15 | 5 min |
| 5 | 1 | 1.75 | 2.25 | 10 min |
| 5 | 1.2 | 4 | 4 | gradual gelation during 24 hours standing |
| 5 | 1.1 | 2 | 2 | |
| 5 | 1.2 | 2 | 2 | |

The mixing of the double alkoxide and triethanolamine is exothermic. This mixture was cooled to ambient temperature before the mixture of water and isopropanol was added.


Baco, Manalox, Aliso and Sardamag are Trade Marks.

CLAIMS

1.      A method of preparing a rigid coherent gel, such method comprising the step of hydrolysing an aluminium alkoxide or aroxide to form a hydrolysate and causing or allowing the hydrolysate to set solid to form the gel.

2.      A method as claimed in Claim 1 wherein the hydrolysate is caused to set by admixture with a basic material.

3.      A method as claimed in Claim 2 wherein the base is a strong organic base, for example, an aminoalcohol.

4.      A method as claimed in any of the preceding claims wherein the alkoxide or aroxide is of aluminium and another metal such as magnesium.

5.      A method as claimed in any of the preceding claims wherein the alkoxide is a propoxide or a butoxide.

6.      A method of preparing a refractory object which comprises mixing a refractory powder with the hydrolysate of any of the preceding claims and causing the slurry thereby produced to set to a desired shape.

7.      A method as claimed in Claim 6 wherein
the refractory powder is magnesia based.